# EUROPEAN PATENT APPLICATION

(11) **EP 0 522 805 A1**
(43) Date of publication of application: **13.01.1993**
(21) Application number: 92306158.4
(22) Date of filing: 03.07.1992
(51) Int. Cl.: G01N 33/18

(54) **Vehicle mounted apparatus and method for determining suitability of effluent water for treatment for reuse**

(30) Priority: 08.07.1991 US 726846
(71) Applicant: NALCO CHEMICAL COMPANY, Naperville Illinois 60563-1198 (US)
(72) Inventor: Latura, William D., Bloomingdale,IL 60108 (US); Voruz, Theodore A., Naperville, IL 60565 (US); Glazer, Abram R., Lisle, IL 60532 (US)
(74) Representative: Harrison, David Christopher

(57) **Abstract**

Apparatus and method for practice in or on a land vehicle to determine whether effluent water released in an operating water treatment system (plant installation) can be adequately treated to remove contaminants so the effluent may be reused at the plant rather than discharged to the environment.

## Description

This invention relates to vehicle mounted apparatus for auditing the water effluent stream from a water treatment system. Typical effluent streams to be analyzed would be derived from process waters contaminated by undesirable components. The effluent may be one presently intended for discharge to the environment, but which, due top increasingly stringent environmental standards, must be prepared for other means of disposal.

Under the present invention, the effluent is audited on-site to determine if it is susceptible to treatment to reduce the contaminants to such levels that the effluent may be returned to the system for reuse.

While mobile systems for an on-site audit of heat exchange efficiency have previously been proposed (Patent Nos. Re. 33,346 and Re. 33,468), there has to the best of our knowledge been no audit apparatus subscribing to the following conditions and arrangement.

The apparatus of the present invention includes holding or equalizing tanks for the effluent sampled on-site and for samples treated under the present invention, means to transfer a sample to functionally different water treatment units for separation of contaminants in the form of solids, destruction of unwanted organics and nutrient support for low plant or animal life which thrive on unwanted organics.

The apparatus includes a laboratory where grab samples of the effluent are initially tested to determine a treatment protocol, to analyze the results of any treatment (unit or combined treatments) and to determine if there is indeed a feasible treatment program.

The apparatus includes a process simulator (pilot plant installation) which simulates a plant water cooling tower or boiler.

A water cooling tower is shown in U. S. Patent No. 4,783,314, the disclosure of which is incorporated herein by reference. A boiler is shown in US Patent No. 5041386, published 20 August 1991, and the disclosure is also incorporated herein by reference.
Fig. 1 is an elevation of a semitrailer in which the present apparatus may be embodied;
Fig. 2 is a diagram illustrating principles of the invention;
Fig. 3 is a plan view of the equalizing or holding tanks and the arrangement of manifolds by which the contents of the holding tanks may be pumped to or received from one of the treating units, one or both simulation pilots and the water used in the laboratory; and
Fig. 4 is a plan view of the treating units, the pilot installations, the laboratory within the vehicle and the manifolds and connectors identified with the tanks shown in Fig. 3.

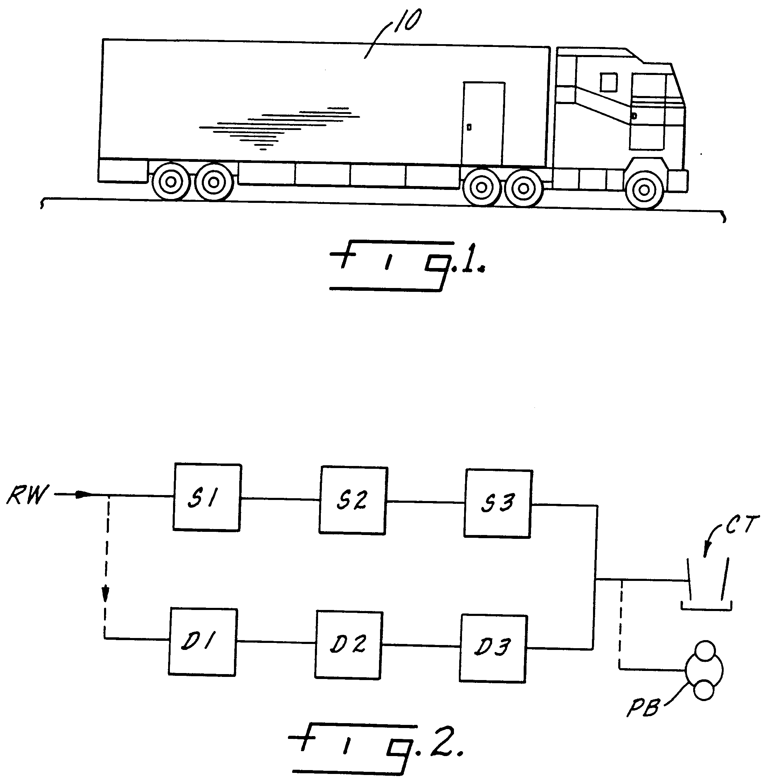

A vehicle which can be employed in the practice of the present invention is shown in Fig. 1 as a large van or semitrailer 10. Holding or flow equalizing tanks (as will be described) are removably supported beneath the frame of the trailer. Water treatment units, functionally of different kinds, are housed within the trailer above the tanks along with analyzers (as will be described) for determining the quality of the water so treated. Process simulation apparatus, either a pilot water cooling tower or pilot boiler, is also staged in the trailer so that a corresponding plant process may be run on a real time basis, using the treated water.

The vehicle will be moved from one plant site to another where effluent water samples at the plant sites are withdrawn, passed to a selected one of the holding tanks and pumped from the tank to a selected one of treatment units where the effluent water is treated to remove the contaminants. The treated water is analyzed to determine if it is feasible to treat the effluent water, or if stronger treatment is required, so that the effluent may be recirculated or otherwise used at the plant site rather than being discharged to the environment.

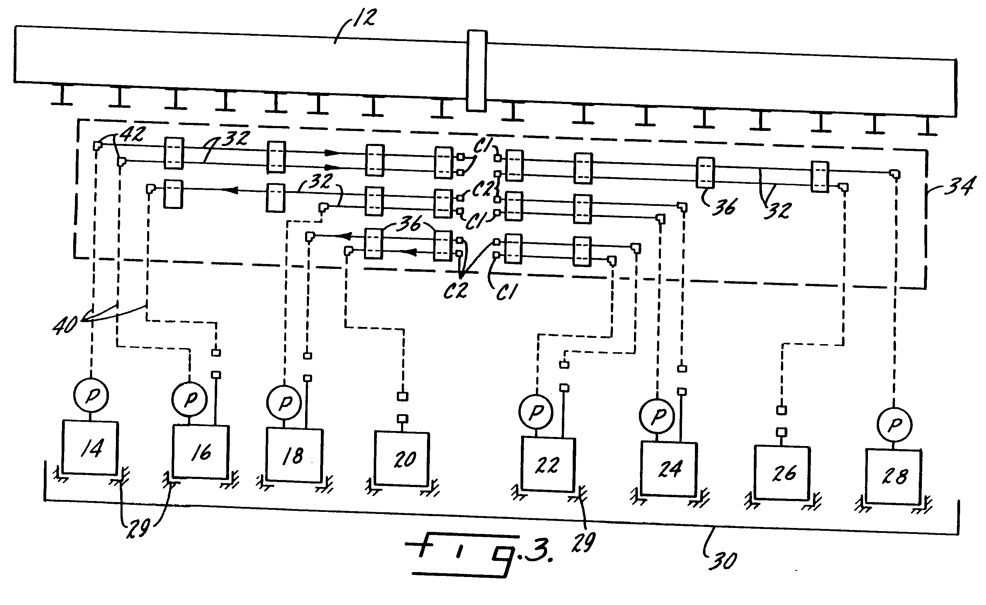

The general arrangement is introduced by what is shown schematically in Fig. 2. The effluent or raw water to be analyzed, RW, is subjected to either a separation process (treating units S1, S2, S3 and so on; as filters or clarifiers) and/or the effluent is subjected to a destructive process to eliminate unwanted organics such as amines, hydrocarbons, phenols and so on (D1, D2, D3 and so on; treatment by oxidation, osonation, pH control, peroxides, ultraviolet light).

The apparatus, Fig. 2, may be employed in different ways, successive treatments S1 and S2, or D1 and D3; or the treatments may cross succession, viz. S1, D2, S3.

In any event after one treatment is completed, say S1, the sample so treated will be returned to a tank and its contents as a second sample then pumped to a second treating unit. Thus, in the simple case of two separation treatments one is undertaken, say S1, Fig. 2, the contents after treatment are returned to a tank, and its contents then pumped from that tank to the second separation treatment unit, S2. Likewise, if the program is destructive of organics, D1 and D2.

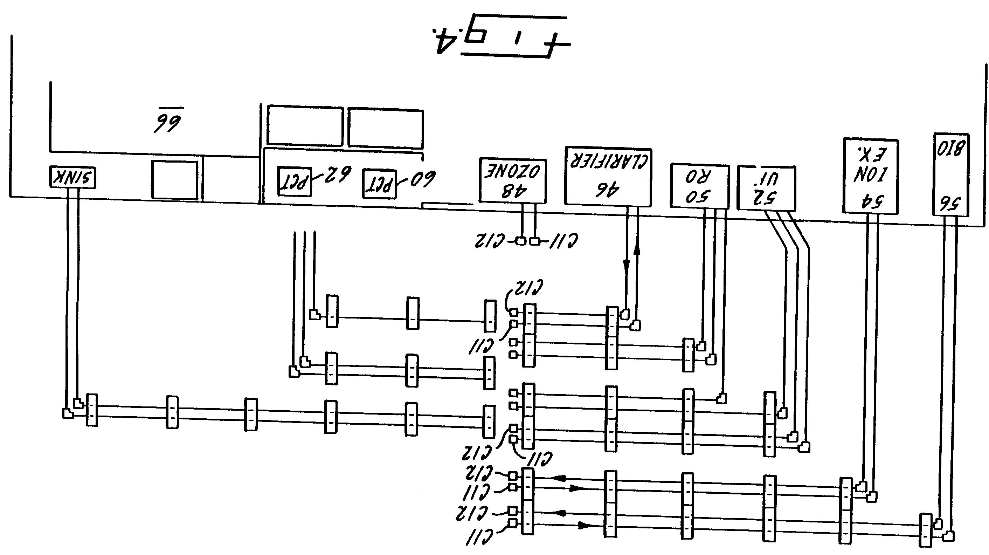

In Fig. 3 the trailer bed is indicated at 12. The equalizing tanks referred to above for receiving the contaminated effluent sample to be transmitted to a treating unit, and for receiving water treated within the vehicle, are identified by reference characters 14, 16, 18, 20, 22, 24, 26 and 28.

As will be explained below, the apparatus may include evaluation apparatus for evaluating characteristics of the water treated by a treating unit, and such evaluating apparatus may include a pilot cooling tower or a pilot boiler CT and PB respectively, Fig. 2. Equalizing tanks 22 and 24 are assigned to the evaluating equipment.

Tanks 26 and 28 are laboratory dedicated for receiving waste water and supplying potable water, respectively.

All tanks are removably supported on guides 29 and of course are locked in place above a water sealed sump 30. After a particular plant site is audited, the tanks are emptied and rigorously cleaned.

An arrangement of lower manifolds or conduits 32 are strung beneath the trailer frame (collectively identified by reference character 34) and are secured in place by clamps 36 to the underside of the vehicle frame. The outlets of the pumps are manually connected by braided hoses as 40 to elbows 42 at the receiving end of the related manifold and each such manifold terminates at the underside of the trailer frame in a connector C1 for transmitting a sample from a tank to a paired connector C11, Fig. 4.

For example, the flexible braided hose which is identified with the clarifier 46 will be joined to its connector C11, Fig. 4, so that the sample effluent in tank 16, Fig. 3, may be pumped to the clarifier 46.

Thus, each of the tanks 16 and 18 for example has a manifold outlet connector C1, Fig. 3, for passing an effluent sample to a treating unit connector C11, Fig. 4, which leads the sample to a treating unit, and in like manner there will be a connector C12, Fig. 4, for returning the treated water to a tank. It is the intention of this disclosure to indicate this uniformity of selection possibilities (C1 to C11) between the various holding tanks and the treating units 46, 48, 50, 52, 54 and 56 shown in Fig. 4. Likewise as return (C12 to C2) from a treating unit back to a tank. A specific example will be given below in which the effluent treated at the clarifier 46, received from tank 16, may be returned to another tank as 14 and the contents then pumped therefrom to the ultrafine filter 52. The clarifier separates large particles (usualy larger than 0.5 microns) while unit 52 separates particles which takes out solids of colloidal size in the range of 0.05 to 0.1 microns.

Thus, the clarifier 46 and the ultrafine filter 52 represent separation treatments. The treatment is a coagulant. Separation is equally true of treating unit 50 (RO, "reverse osmosis" which may be employed to remove dissolved contaminants or for sea water desalinization). Treating unit 50 thus injects, selectively, scale inhibitors, pH controllers and biocides. The ion exchange treating unit 54 is employed for removal of soluble ionic species. Strong acid, weak acid, strong base and weak base resins are employed. It embodies an absorption column configuration.

In comparison, the ozone treating unit 48 is another destruction unit in which inimical organics are destroyed, selectively, by osonation or by ultraviolet light, or by peroxide or by pH control or any combination.

Treating unit 56 is employed to feed nutrients to low plant life which also destroy unwanted organics such as hydrocarbons.

Again, a particular treatment and analysis procedure may be as follows.

### Example 1 - Separation

(1) the raw effluent will be transferred from the plant site to tank 14, partially filling the tank.
(2) The pump P identified with tank 14 will transfer the effluent to the clarifier 46 by way of the related braided hosing 40, elbow 42, manifold 32 and finally through the braided hosing which joins connectors C1 and C11 leading to the input of the clarifier 46.
(3) Following the clarification treatment (for example treatment by a flocculent) a sample of the result will be taken and analyzed for effectiveness of the treatment in the laboratory in the vehicle, as hereinafter described.

Regardless of the efficacy of the clarification treatment, the contents so treated are returned to tank 16 by way of connection C12 (from the clarifier) to connection C2 which leads back to tank 16.

In all instances (tanks 14, 16, 18) connectors C1 and C11 transfer from a tank to a treating unit; connectors C2 and C12 are in the return lines back to a tank.

### Example 2 - Destruction

1. Use either tank 14 or 16;
2. Transfer to 48 then back to tank;
3. If further treatment at 56 is needed, pump from tank 56 and return to tank.

### Example 3

Examples 1 and 2 are combined so that both separation and destruction are applied to the sample.

The laboratory with the vehicle will report the proposed sequence (configuration) of unit treatments configured to the laboratory analysis of the effluent water. This report or recommendation will be based on an initial analysis of the plant effluent.

The configured results are stored in one of the tanks 22, 24 and the water so treated is then employed as the source water for a pilot cooling tower (or pilot boiler) within the vehicle. There are two pilot cooling towers 60 and 62, Fig. 4, so that alternate configurations resulting from a treatment sequence may be analyzed in terms of the pilot effluent for efficacy of the programmed treatment configuration. Thus, treated water emergent from the last step of a treatment configuration or sequence may be stored in tank 24 for use at pilot 60 and a second treatment sequence stored in tank 26 for use at pilot 62. As shown in Fig. 2, the pilot cooling towers may be replaced by pilot boilers.

Referring to Fig. 4, the general area of the laboratory is designated by reference character 66. The analysis equipment may include a computer, an atomic adsorption spectrometer, chemical oxygen demand reactor, pH and conductivity analyzers and biological oxygen demand analyzers.

The effluent from the on-site plant is to be subjected to treatment to determine if it can be so treated to be of a quality for reuse at the plant site which may be characterised by a water cooling tower or boiler. The laboratory supervisor will suggest a protocol or treatment sequence after analyzing the raw effluent. The protocol or treatment configuration may involve separation of contaminants, destruction of contaminants or both. There may be successive separations involving different species of treatment; equally so as destruction of contaminants; between successions, the sample, always an effluent but partly treated, is returned to a tank and pumped to the new treating unit featured in the treatment configuration. If it is determined by laboratory analysis the effluent can be revitalized as a useful source of water, the final sample is transmitted to a tank and from thence is pumped to the pilot simulator to be subjected to replication of the plant process. The water so employed as a replicate is finally analysed to assure its efficacy, that is, water of a purity which can be indeed by reused at the plant.

## Claims

1. Apparatus contained in or on a land vehicle to determine whether effluent water can be adequately treated to remove contaminants to an acceptable level for reuse, comprising a plurality of tanks, a plurality of functionally different water treatment units into which an effluent sample and or treated water sample can be selectively pumped for treatment to reduce the contaminant level, means for selectively introducing samples of plant effluent water and.or treated water into the tanks and the units, and analyzers for analyzing an original effluent sample and a treated sample to determine the initial level of contaminant and contaminant level after treatment.

2. Apparatus according to claim 1 in which the treating units include at least a pair of course and fine solid separation treating units connected in series through one of the equalizing tanks, and in which the pumps, manifold and hoses are so connected to successively separate coarse and fine particulate contaminants in a series treatment.

3. Apparatus according to claim 1 or claim 2 in which one of the treating units is employed to destroy organic contaminants in the sample as by oxidation or ultraviolet light amid in which a second treating unit feeds nutrients to the sample for any low life forms therein, and in which said first and second treating units are connected in series through one of the equalizing tanks.

4. Apparatus according to claim 1 including: a first treating unit (a) for separating solid contaminants in a sample and a second treating unit (b) for feeding nutrients to low life forms in the sample; and in which a sample can be pumped from one equalizing tank to one of the treating units (a) or (b), returned to an equalizing tank and pumped therefrom to the other treating unit.

5. Apparatus according to any of the above claims including process simulation apparatus within the trailer adjacent the treatment units capable of evaluating the performance characteristics of the treated water in an end use application which simulates operation of said plant, and in which treated water can be fed thereto for performance evaluation.

6. Apparatus according to claim 5 in which the simulation apparatus is either a pilot cooling tower or a pilot boiler.

7. A method of determining if effluent water can be adequately treated to remove contaminants for reuse comprising the following steps performed in or on a land vehicle.
(1) transferring a sample of the effluent to one or more tanks;
(2) introducing an effluent sample into one of several water treatment units;
(3) returning the sample as a treated sample to one of the tanks; and
(4) repeating step (2) and step (3) with the treated sample for further treatment if contaminants in the treated sample are determined as not reduced to a level where the effluent water is reusable.

8. A method according to claim 7 in which (if the treated sample is determined as having a contaminant level low enough for the plant effluent to be reused when so treated) the treated sample is employed as water in a pilot cooling tower or pilot boiler inside the vehicle as a final determination of efficacy for reuse at the plant.
